**Europäisches Patentamt**

**European Patent Office**

**Office européen des brevets**

(19)

(11) Publication number: **0 229 994
B1**

(12)

# EUROPEAN PATENT SPECIFICATION

(45) Date of publication of the patent specification:
**03.05.89**

(51) Int. Cl.⁴: **C07C 7/13,** C07C 11/02,
C07C 41/36

(21) Application number: **86117398.7**

(22) Date of filing: **13.12.86**

(54) Process for the removal of dimethyl ether contained as an impurity in liquid olefinic C3-C5 feeds.

(30) Priority: **16.12.85 US 809226**

(43) Date of publication of application:
**29.07.87 Bulletin 87/31**

(45) Publication of the grant of the patent:
**03.05.89 Bulletin 89/18**

(84) Designated Contracting States:
**AT BE CH DE ES FR GB GR IT LI LU NL SE**

(56) References cited:
**No relevant documents have been disclosed**

(73) Proprietor: **UNION CARBIDE CORPORATION, 39 Old
Ridgebury Road, Danbury Connecticut 06817(US)**

(72) Inventor: **Nagji, Moez Mohammedali, 24 First Street,
Yonkers New York 10704(US)**
Inventor: **Corvini, Giacomo, 165 Route 37, New Fairfield,
CT 06812(US)**

(74) Representative: **Eggert, Hans-Gunther, Dr.,
Räderscheidtstrasse 1, D-5000 Köln 41(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates in general to the removal of dimethyl ether as an impurity from olefinic feedstocks, and more particularly to the liquid phase purification of olefinic feedstocks wherein the major proportion of olefinic hydrocarbons contains from four to five carbon atoms and the dimethyl ether impurity is present in amounts of from 1 to 5000 ppm (wt.), said purification being accomplished by selective adsorption of the dimethyl ether on a crystalline zeolite molecular sieve having the faujasite type of crystal structure. The invention also relates to a process for preparing alkyl tertiary alkyl ethers wherein the present purification process is employed to remove dimethyl ether from the olefinic hydrocarbons reactants used therein.

The use of zeolitic molecular sieves to separate mixtures of molecular species into constituent components based on molecular size and/or degree of polarity is a well-established practica and finds numerous present commercial applications. For example in our copending European application No. 86 900 435.8 (EP-A 205 562) zeolitic molecular sieves, including types A, X and Y, are empolyed to selectively adsorb methanol from admixture with $C_4$ hydrocarbons and dimethyl ether in the course of recovering methanol for recycle to a reactor in the preparation of methyl tert.-butyl ether (MTBE).

It is also known, however, that a number of factors influence the suitability of a particular molecular sieve for adsorbing a particular molecular species from admixture with other molecular species, so that it is not possible to accurately predict the commercial feasibility of an intended separation. For example, both qualitative and quantitative changes in the cation population of a zeolite adsorbent can have a marked effect of its adsorptive behavior with respect to a particular adsorbate, but the results are entirely unpredictable as demonstrated in U.S.P. 3 885 927. In another instance it was found that the capacity of a zeolitic adsorbent for a particular impurity adsorbate was unpredictably increased if the zeolite was first contacted by a fluid containing the impurity which was more strongly adsorbed than the fluid of a second feedstock containing the same impurity which subsequently was passed over the zeolite without intervening desorption. Such a phenomenon is elucidated in the disclosure of U.S.P. 3 660 967.

Further, there is the matter of the catalytic activity of the zeolite adsorbent toward one or more of the constituents of feedstock passed over the zeolite for purification purposes. A common problem with undesirable catalytic activity occurring in an adsorption process utilizing zeolitic molecular sieves involves the dehydration and sweetening of natural gas streams which also contain carbon dioxide. It is observed that whereas essentially all commercially available zeolites are capable of adsorbing both water and $H_2S$ from natural gas, some, notably zeolite 5A, are much more effective in removing sulfur than others such as zeolite 13X. The reason is that zeolite 5A does not unduly catalyze the reaction of $H_2S$ with $CO_2$ to form COS, whereas zeolite 13X exhibits very strong catalytic activity in this regard. This difference is evident from the following data obtained during the fixed-bed purification treatment of city gas containing 2.2 volume percent carbon dioxide and either 33 or 40 ppm (vol.) hydrogen sulfide by contact with zeolite 5A, zeolite 4A and zeolite 13X.

| Zeolite | Feed $H_2S$ | Temp. | $H_2S$ Loading on Zeolite, [a] lb/100 lb Adsorbent | | $H_2S$ Converted to COS, % |
|---------|-------------|-------|------------|-----------|-------------|
| | | | Apparent [b] | Actual [c] | |
| 5A | 40 | 76 | 0.16 | 0.15 | 5 |
| 4A | 33 | 60 | 0.93 | 0.42 | 55 |
| 13X | 33 | 60 | 1.07 | 0.13 | 88 |

(a) When bed effluent reached an $H_2S$ content of 1 ppm (v.)
(b) Includes $H_2S$ adsorbed plus converted to COS.
(c) Does not include $H_2S$ converted to COS.

In the foregoing example of catalytic activity exhibited by molecular sieve adsorbents, it does not appear that the micropore volume of the zeolite crystal is a factor in the production of COS. Prior to the present invention, however, it was observed that the presence of methanol in a natural gas stream being dried and sweetened by passage through a fixed-bed of zeolite 5A adsorbent caused the excessive and rapid build-up of deactivating coke deposits on the adsorbent. The accepted theory was that the phenomenon required a relatively large micropore volume of the zeolite present, and that the pores of zeolite 5A were sufficiently large to accommodate the following series of reactions:

$2CH_3OH \rightarrow CH_3\text{–}O\text{–}CH_3 + H_2O$ (I)

$n\ CH_3\text{–}O\text{–}CH_3 \rightarrow C_2$, to $C_5$ Olefins + $H_2O$ (II)

Reaction (I), an etherfication reaction, produces a relatively large ether molecule from the smaller methanol molecule, and since the reaction proceeds within the micropores of the zeolite, the reaction is facilitated by the larger pore volume of zeolite 5A compared with the somewhat smaller pore volume of zeolite 4A. Once formed the dimethyl either can undergo a zeolite-catalyzed dehydration reaction to form olefins which polymerize and then thermally degrade to cause the undesirable coke deposition and adsorbent deactivation. It was generally perceived that larger unit cell pore volume adsorbants such as zeolite 13X and macroporous adsorbents, such as alumina and silica gel, would favor formation of dimethyl ether, and were thus to be avoided in processes of the type examined.

It has now been discovered that dimethyl ether can be selectively removed as an impurity component from an olefinic hydrocarbon feedstock by the process which comprises passing said feedstock consisting essentially of a liquid light hydrocarbon stream containing from 5 to 50 weight percent monolefins having from 3 to 5 carbon atoms, from 100 to 20,000 ppm by weight diolefins, and also containing from 1 to 5,000 ppm, preferably 50 to 3,000 ppm by weight of dimethyl ether, in liquid state through an adsorbent mass of a crystalline zeolite molecular sieve having the crystal structure of faujasite at a temperature of from 0°C to 60°C and at a pressure of about 2.04 to 35.46 bar preferably 4.46 to 28.57 bar, whereby the dimethyl ehter is selectively adsorbed and retained on the adsorbent mass and a purified hydrocarbon effluent is recovered, periodically terminating the passage of said feedstock through the adsorbent mass and at least partially regenerating the said adsorbent by purging with a fluid stream of hydrocarbons or inert gases to desorb dimethyl ether therefrom, and thereafter again passing the olefinic feedstock in the liquid state through the adsorbent mass to obtain purified hydrocarbon effluent. By the term liquid light hydrocarbons, as used herein, is meant hydrocarbon molecular species which are in the liquid state at 23°C and one atmosphere pressure and which contain six carbon atoms or less. Such hydrocarbons include paraffins, olefins, diolefins, acetylenes, and aromatically unsaturated species such as benzene. Optionally, the liqid light hydrocarbon stream can contain relatively small amounts, i.e., less than about 2000 ppm (wt.) of oxygen-containing materials such as water, methanol and higher alcohols, and ethers other than dimethyl ethers, such as methyl tert.-butyl ether, having up to six carbon atoms. Preferably the feedstock is a mixed $C_{4-5}$ hydrocarbon stream from a petroleum refinery operation such as a $C_{4-5}$ stream from a fluidized catalytic cracking (FCC) process from which, in either case, the bulk of the isobutylene and/or isoamylene content has been removed by conversion to MTBE or TAME, respectively, by reaction with methanol.

The zeolite adsorbent can be any naturally-occurring or synthetically prepared crystalline zeolite having the faujasite-type of crystal structure, including zeolite X disclosed in U.S. Patent 2 882 244; zeolite Y disclosed in U.S. Patent 3 130 007; and zeolite LZ-210 disclosed in U.S. Patent 4 503 023. The mineral faujasite is well-known, but as yet has not been found in commercial quantities. By the term zeolite as used herein is meant not only compositions having framework structures formed from $AlO_2$- and $SiO_2$ tetrahedra, but also those in which the framework structures include tetrahedral oxide units containing one or more of phosphorus and metals having valence values, of +2, +3 or +4. The zeolite adsorbent is preferably employed as either self bonded agglomerates or pellets or agglomerates bound with conventional binders such as haolinitic or attapulgus clays.

The adsorption system is not a critical factor and can be either fixed bed, moving bed, simulated moving bed or fluidized bed, but is preferably of the fixed-bed type. Since the feedstock containing the dimethyl ether is treated by the present process in the liquid phase, the temperature must be below the critical temperature of the feedstock constituents and is preferably in the range of 0°C to 50°C. Pressure conditions are selected with references to the temperature conditions to ensure liquid phase operation during the adsorption-purification phase of the process.

For carrying out the periodic regeneration of the adsorbent, vapor phase regeneration mode is employed using a purge gas at a temperature higher than the temperature during the previous adsorption stage, preferably a temperature within the range of 150°C to 300°C. Since the previous adsorption stage was carried out in the liquid phase, the adsorption bed void space contains liquid feedstock at the time of termination of adsorption, and this void space liquid is preferably drained from the bed prior to the commencement of the adsorbent regeneration. The purge gas utilized is not a critical factor, and can be either non-absorbable or sorbable as those terms are understood in the art. The non-absorbable gases include nitrogen, hydrogen, helium and methane. The sorbable gases should be hydrocarbons essentially free of olefinic and acetylenic unsaturation because of the tendency of such compounds to thermally degrade in contact with molecular sieve adsorbents at elevated temperatures, so that upon return of the regenerated adsorbent bed to the adsorption-purification stage of the present process, the capacity to adsorb and sequester dimethyl ether from the feedstock is significantly reduced. Preferably saturated hydrocarbons having from 1 to 5 carbon atoms are employed as purge gas materials, with n-butane being especially preferred. Not only is n-butane commonly available at petroleum refinery sites, but it is adsorbed on the faujasite-type molecular sieve to an optimal degree, and is also frequently used as a blending agent for gasoline. The dimethyl ether which it adsorbs is also acceptably present in gasoline and, accordingly, no separation of n-butane from the desorbed dimethyl ether need be made as would be necessary if separate uses were to be made of the ether and the purge gas.

As is well known in the art, it is frequently advantageous to pre-load a zeolite adsorbent with an easily desorbably adsorbate prior to contact with a feedstock to be purified in order that the heat of adsorption of the adsorbates from the feedstock is in part countered by the heat of desorption of the pre-loaded adsorbate. This results in the reduction in magnitude of the heat front in fixed adsorption beds and the consequent reduction is adsorbate loading and destabilizing activity occurring when large heat fronts progress through the bed during the adsorption-purification stage of the process. Thus when the adsorption bed of the present process is regenerated using a non-absorbable purge gas, there is created an adsorbent mass in a highly activated state, which upon contact with new feedstock can create an undesirably large heat front. To ameliorate this phenomenon, it is advantageous to cool the newly regenerated bed and pre-load the adsorbent with an adsorbate, such as a $C_2$–$C_4$ alkane, which does not interfer with the adsorption of the dimethyl ether during the following adsorption-purification stage.

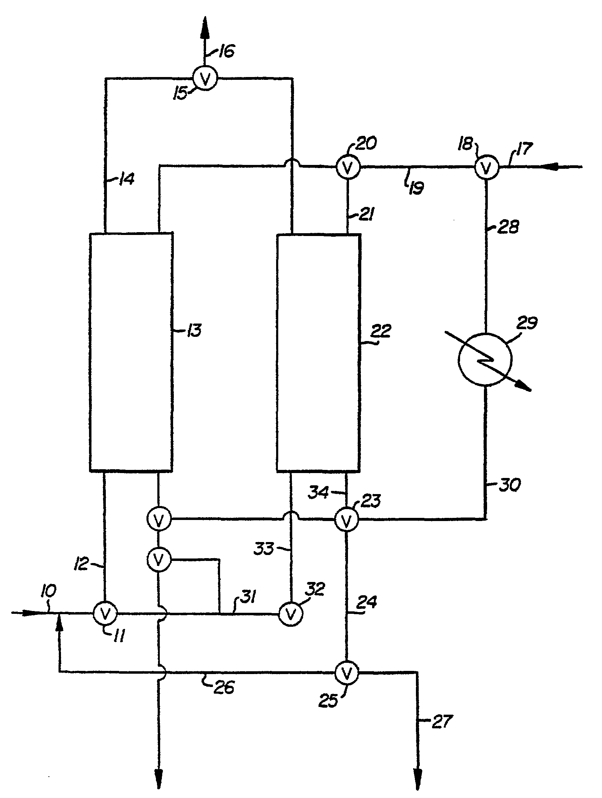

The drawing is a schematic flow diagram illustrating one embodiment of the generic process of the present invention.

With respect to the drawing, the process embodiment involves the use of a system containing two fixed-bed adsorption units operated in a continuous manner whereby one bed is engaged in the purification of feedstock while the other bed is undergoing the steps of hot-purge regeneration, cool-down and preloading. In the interest of simplifying the drawing, the flow diagram omits pumping and heating means, the necessity and location of which are obvious to those skilled in the art. The two adsorption beds, 13 and 22, contain equal quantities of clay-bonded 1.59 mm extruded pellets of crystalline sodium zeolite X. A liquid phase feedstock having the following compositions

| Constituent | Weight-% |
|---|---|
| $H_2$ | 0.003 |
| Ethane | 0.008 |
| Propane | 1.165 |
| Propylene | 0.627 |
| n-Butane | 0.14 |
| i-Butane | 35.06 |
| Butyne-1 | 16.37 |
| iso-Butylene | 0.55 |
| trans-Butene | 26.66 |
| cis-Butene | 17.85 |
| iso-$C_5$ | trace |
| n-$C_5$ | trace |
| $C_{6+}$ | trace |
| 1–3, Butadiene | 0.014 |
| MeOH | 1.46 |
| $H_2O$ | 0.09 |
| Demethylether | 0.03 |

is fed into the system, at a temperature of about 20°C, through line 10, valve 11 and line 12 to the bottom of adsorption bed 13. The absorbent in bed 13 contains a loading of n-butane of about 10 weight percent as a result of the previous regeneration and cool-down using n-butane. The DME and other strongly adsorbable constituents of the feedstock are adsorbed on the bed with the passage of feedstock thereinto with the relatively non-adsorbed paraffins flowing from bed 13 through line 14, valve 15 and leaving the system through 16 during even the early stages of the adsorption-purification step. Prior to breakthrough of dimethyl ether from bed 13, the feedstock is diverted to bed 22 through line 31, valve 32 and line 33, and bed 13 is regenerated in the same manner as described hereinafter with respect bed 22. At the beginning of the regeneration, bed 22 contains a loading of about 1 weight percent dimethyl ether on the zeolite X adsorbent and the bed void space contains essentially the same liquid composition as the feedstock being treated. In the initial stage of the regeneration, the void-space liquid is drained from bed 22 through line 34, valve 23, line 24, valve 25 and is passed either out of the system through line 27 or combined with the incoming feedstock in line 10, as desired. The void-space drawing can be solely by gravity, or can be expedited by the inflow into the opposite end of the bed of a vapor phase stream of n-butane at a temperature of about 200°C. entering the system at line 17, passing through valve 18, line 19, valve 20, and line 21. When bed 22 is essentially free of void-space liquid, the n-butane entering the bed through line 21 is raised in temperature to about 250°C to facilitate desorption of the dimethyl ether. The bed effluent is passed through line 34, valve 23, line 24, valve 25 and out of the system through line 27. At the end of the desorption stage, the bed is at essentially the same temperature as the n-butane purge gas, i.e., about 250°C. The bed is then cooled by passing n-butane at a temperature of about 25°C countercurrently, with respect to the direction of flow during the preceding purge-desorption stage, through the bed through the closed loop consisting of valve 18, line 28, cooler 29, line 30, valve 23, line 34, bed 22, line 21, valve 20 and line 19. Initially, the n-butane entering the hot bed is vaporized, but as the temperature of bed 22 is lowered, vaporization ceases and ultimately liquid butane fills the bed, at which time n-butane flow is terminated and the flow of liquid phase feedstock through line 33 is begun to initiate the adsorption-purification stage of the process.

In a preferred embodiment of the present process, the feedstock containing the dimethyl ether is a hydrocarbon mixture derived from the preparation of methyl tert.-alkyl ether and the adsorption-purification procedure is utilized as an adjunct thereto so that the $C_4$–$C_5$ hydrocarbons which are not reacted with methanol to form the desired methyl tert.-alkyl ethers can be purified of dimethyl ether, and also MTBE, water and small amounts of methanol, for use as an alkylation feedstock. Thus in the preferred process, dimethyl ether is removed as an impurity component from an olefinic hydrocarbon feedstock containing from 1 ppm to 5000 ppm (weight) dimethyl ether by passing said feedstock in the liquid state through an adsorbent mass of a crystalline zeolite molecular sieve having the crystal structure of faujasite at a temperature of from –40°C to 150°C, preferably 0°C to 40°C, and at a pressure sufficient to maintain the feedstock in the liquid phase whereby the dimethyl ether is selectively adsorbed and retained on the adsorbent mass and a purified hydrocarbon effluent is recovered, said olefinic hydrocarbon feedstock being generated by the process which comprises the steps of:

(a) contacting and reacting in the liquid phase a reaction mixture formed by combining a stream consisting essentially of hydrocarbons having from 4 to 5 carbon atoms and containing at least some proportion of an isoalkylene having from 4 to 5 carbon atoms with a stoichiometric excess of methanol, with respect to the isoalkylene, to form a reaction product comprising methyl tert.-alkyl ether, unreacted methanol and unreacted $C_4$–$C_5$ hydrocarbons and dimethyl ether;

(b) isolating the methyl tert.-alkyl ether from the reaction product by distillation;

(c) reducing the unreacted methanol content of the residual portion of the reaction product to less than about 1000 ppm by weight, preferably by selective adsorption thereof to provide said olefinic hydrocarbon feedstock.

A process for preparing alkyl tertiary alkyl ethers with produces a preferred feedstock for use in the present invention is described in copending European application No. 86 900 435.8 (EP-A 205 562) and in U.S. Patent Nos. 3 726 942; 4 371 718; 4 490 563 and 4 504 688; the entire disclosures of which is incorporated herein by reference. A typical olefinic hydrocarbon feedstock derived from such processes prior to methanol removal can have the composition:

| Component | Wt.-% |
|---|---|
| $H_2$ | 0.002 |
| Ethane | 0.007 |
| Propane | 1.02 |
| Propylene | 0.55 |
| n-Butane | 0.124 |
| i-Butane | 30.77 |
| Butyne-1 | 14.37 |
| i-Butylene | 0.48 |
| trans-Butene | 23.39 |
| cis-Butene | 15.66 |
| iso-$C_5$ | 0.001 |
| n-$C_5$ | <0.001 |
| $C_{6+}$ | <0.001 |
| 1,3-Butadiene | 0.013 |
| MeOH | 1.277 |
| $H_2O$ | 0.078 |
| Dimethylether | 0.027 |

## Claims

1. Process for the removal of dimethyl ether as an impurity component from an olefinic hydrocarbon feedstock which comprises passing said feedstock consisting essentially of a liquid light hydrocarbon stream containing from 5 to 50 weight percent monolefins having from 3 to 5 carbon atoms, from 100 to 20,000 ppm by weight diolefins, and also containing from 1 to 5000 ppm by weight of dimethyl ether, in liquid state through an adsorbent mass of a crystalline zeolite molecular sieve having the crystal structure of faujasite at a temperature of from 0°C to 60°C and at a pressure of about 2.04 to 35.46 bar whereby the dimethyl ether is selectively adsorbed and retained on the adsorbent mass and a purified hydrocarbon effluent is recovered, periodically terminating the passage of said feedstock through the adsorbent mass and at least partially regenerating the said adsorbent by purging with a fluid stream of a hydrocarbon or an inert gas to desorb dimethyl ether there from, and thereafter again passing the olefinic feedstock in the liquid state through the adsorbent mass to obtain purified hydrocarbon effluent.

2. Process according to claim 1 wherein the dimethyl ether content of the feedstock being treated is from about 50 to 3000 ppm.

3. Process according to claim 2 wherein the fluid stream used to at least partially regenerate the adsorbent mass is n-butane.

4. Process according to claim 3 wherein the adsorbent employed to selectively adsorb dimethyl ether is zeolite X or zeolite Y.

5. Process according to claim 1 wherein liquid light hydrocarbon feedstock being treated for removal of dimethyl ether is produced by the process which comprises the steps of:

(a) contacting and reacting in the liquid phase a reaction mixture formed by combining a stream consisting essentially of hydrocarbons having from 4 to 5 carbon atoms and containing at least some proportion of an isoalkylene having from 4 to 5 carbon atoms with a stoichiometric excess of methanol, with respect to the isoalkylene, to form a reaction product comprising methyl tert.-alkyl ether, unreacted methanol and unreacted $C_4$–$C_5$ hydrocarbons and dimethyl ether;

(b) isolating the methyl tert.-alkyl ether from the reaction product by distillation; and

(c) reducing the unreacted methanol content of the residual portion of the reaction product to less than about 1000 ppm by weight.

## Patentansprüche

1. Verfahren zur Entfernung von Dimethylether als Verunreinigungskomponente aus einem olefinischen Kohlenwasserstoff-Speisestrom, umfassend das Hindurchleiten dieses Speisestroms bestehend im wesentlichen aus einem Strom leichter flüssiger Kohlenwasserstoffe enthaltend von 5 bis 50 Gew.-% Monoolefine mit 3 bis 5 Kohlenstoffatomen, von 100 bis 20 000 ppm an Gewicht Diolefine und enthaltend außerdem von 1 bis 5000 ppm an Gewicht Dimethylether, im flüssigen Zustand durch eine Adsorbensmasse eines kristallinen zeolithischen Molekularsiebs mit einer Kristallstruktur des Faujasits, bei einer Temperatur von zwischen 0°C und 60°C und bei einem Druck von etwa 2,04 bis 35,46 bar, wobei der Dimethylether selektiv adsorbiert und an der Adsorbensmasse zurückgehalten wird und wobei der gereinigte ausströmende Kohlenwasserstoff gewonnen wird, wobei man periodisch das Durchströmen dieses Speisestroms durch die Adsorbensmasse beendet und das Adsorbens wenigstens teilweise durch Reinigen mit einem Fluidstrom eines Kohlenwasserstoffs oder einem Inertgas regeneriert, um Dimethylether von diesem zu desorbieren, und man danach den olefinischen Speisestrom wiederum im flüssigen Zustand durch die Adsorbensmasse leitet, um gereinigten ausströmenden Kohlenwasserstoff zu erhalten.

2. Verfahren nach Anspruch 1, bei dem der Dimethylethergehalt des behandelten Speisestroms zwischen 50 und 3000 ppm liegt.

3. Verfahren nach Anspruch 2, bei dem der für das wenigstens teilweise Regenerieren der Adsorbensmasse verwendete Fluidstrom n-Butan ist.

4. Verfahren nach Anspruch 3, bei dem das zum selektiven Adsorbieren von Dimethylether verwandte Adsorbens Zeolith X oder Zeolith Y ist.

5. Verfahren nach Anspruch 1, bei dem der Speisestrom flüssiger leichter Kohlenwasserstoffe, der behandelt wird, um Dimethylether zu entfernen, in einem Verfahren hergestellt wird, das die Schritte umfaßt:

(a) in Kontakt bringen und Reagierenlassen einer Reaktionsmischung in der Flüssigphase, die gebildet wird durch Kombinieren eines Stroms bestehend im wesentlichen aus Kohlenwasserstoffen mit 4 bis 5 Kohlenstoffatomen und enthaltend wenigstens einen Anteil eines Isoalkylens mit 4 bis 5 Kohlenstoffatomen mit einem stöchiometrischen Überschuß an Methanol im Hinblick auf das Isoalkylen, zur Bildung eines Reaktionsprodukts des Methyl-tertiär-Alkylether, nicht umgesetztes Methanol und nicht umgesetzte $C_4$–$C_5$-Kohlenwasserstoffe und Dimethylether enthält;

(b) Isolieren des Methyl-tertiär-Alkylethers aus dem Reaktionsprodukt durch Destillation; und

(c) Reduzieren des nicht umgesetzten Methanolgehalts des Rückstandsanteils des Reaktionsproduktes auf weniger als etwa 1000 ppm, bezogen auf das Gewicht.

## Revendications

1. Procédé pour éliminer l'éther de diméthyle en tant qu'impureté d'une charge d'alimentation constituée d'hydrocarbures oléfiniques, qui consiste à faire passer ladite charge d'alimentation essentiellement constituée d'un courant liquide d'hydrocarbures légers contenant 5 à 50% en poids de mono-oléfines ayant 3 à 5 atomes de carbone, 100 à 20 000 ppm en poids de dioléfines, et contenant également 1 à 5000 ppm en poids d'éther de diméthyle, à l'état liquide sur une masse adsorbante d'un tamis moléculaire zéolitique cristallin ayant la structure cristalline de la faujasite à une température de 0°C à 60°C et à une pression d'environ 2,04 à 35,46 bars de manière que l'éther de diméthyle soit sélectivement adsorbé et retenu sur la matière adsorbante et qu'un effluent hydrocarboné purifié soit recueilli, à interrompre périodiquement le passage de ladite charge d'alimentation sur la matière adsorbante et à régénérer au moins partiellement ladite matière adsorbante par purge avec un courant fluide d'un hydrocarbure ou d'un gaz inerte pour en désorber l'éther de diméthyle, puis à faire passer de nouveau la charge oléfinique d'alimentation à l'état liquide sur la matière adsorbante pour obtenir un effluent hydrocarboné purifié.

2. Procédé suivant la revendication 1, dans lequel la teneur en éther de diméthyle de la charge d'alimentation en traitement va d'environ 50 à 3000 ppm.

3. Procédé suivant la revendication 2, dans lequel le courant fluide utilisé par régénérer au moins partiellement la matière adsorbante est du n-butane.

4. Procédé suivant la revendication 3, dans lequel la matière adsorbante utilisée pour adsorber sélectivement l'éther de diméthyle est la zéolite X ou la zéolite Y.

5. Procédé suivant la revendication 1, dans lequel la charge liquide d'hydrocarbures légers en traitement en vue de l'élimination de l'éther de diméthyle est produite par le procédé qui comprend les étapes consistant:

(a) à mettre en contact et à faire réagir en phase liquide un mélange réactionnel formé en associant un courant essentiellement constitué d'hydrocarbures ayant 4 ou 5 atomes de carbone et contenant au moins une certaine proportion d'un iso-alkylène ayant 4 ou 5 atomes de carbone avec un excès stoechiométrique de méthanol, par rapport à l'iso-alkylène, pour former un produit de réaction comprenant de l'éther de méthyle et de tertio-alkyle, du méthanol n'ayant pas réagi et des hydrocarbures en $C_4$–$C_5$ n'ayant pas réagi et de l'éther de diméthyle,

(b) à isoler l'éther de méthyle et de tertioalkyle du produit réactionnel par distillation; et

(c) à réduire la teneur en méthanol n'ayant pas réagi dans la portion résiduelle du produit de réaction à une valeur inférieure à environ 1000 ppm en poids.